# EUROPEAN PATENT APPLICATION

(11) **EP 2 156 855 A1**
(43) Date of publication of application: **24.02.2010**
(21) Application number: 08739721.2
(22) Date of filing: 02.04.2008
(51) Int. Cl.: A61M 1/14

(54) **CONTINUOUS BLOOD PURIFICATION SYSTEM PROVIDED WITH SYRINGE PUMPS**

(30) Priority: 02.04.2007 JP 2007095975
(71) Applicant: Nipro Corporation, Osaka-shi Osaka 531-8510 (JP)
(72) Inventor: UEDA, Mitsutaka, Osaka-shi Osaka 531-8510 (JP); MITSUHASHI, Makoto, Osaka-shi Osaka 531-8510 (JP)
(74) Representative: Bertsch, Florian Oliver
(86) International application number: PCT/JP2008/056611
(87) International publication number: WO 2008/120803

(57) **Abstract**

It is intended to provide a continuous blood purification system which shows extremely small errors in the dialysate flow rate and the replacement fluid flow rate and requires no pump calibration.

The continuous blood purification system according to the present invention comprises a blood purifier (1), a blood circuit (2), a dialysate-supply line (4), a replacement fluid-supply line (5) and a water-removal line (6), wherein the dialysate-supply line (4), the replacement fluid-supply line (5) and the water-removal line (6) are provided with syringe pumps (43), (53) and (62), respectively. With respect to the dialysate-supply line (4), the dialysate which is drawn in at once to the syringe pump (43) can be continuously supplied in small portion to the blood purifier (1). With respect to the replacement fluid-supply line (5), the replacement fluid which is drawn in at once to the syringe pump (53) can be continuously supplied in small portion to the venous side or arterial side of the blood circuit. With respect to the water-removal line (6), the used dialysate which is continuously drawn in to the syringe pump (62) in small portion can be discharged at once.

## Description

### TECHNICAL FIELD

The present invention relates to a continuous blood purification apparatus for purifying blood taken out from a blood vessel to the outside of a human body and supplementing a useful material to the blood if necessary, thereby assisting or carrying out organ function of a living body.

### BACKGROUND TECHNIQUE

A blood purifying therapy such as hemodialysis, hemofiltration or the like has been hitherto carried out to improve critical states of patients who suffer from renal failure or hyperhydration due to chemical grouting after surgery. There has been adopted such an alternative method that a clinician properly determines which one of hemodialysis and hemofiltration should be selected as a medical treatment for a patient while taking the state of the patient into consideration and selects hemodialysis or hemofiltration. Furthermore, the treatment of the blood purifying therapy is generally carried out in a short period of time of about 4 to 6 hours per treatment. However, in the case of a patient who develops complications such as blood poisoning, cardiopulmonary dysfunction or the like, such a short-time blood purifying therapy may bring an adverse effect to the patient because it causes rapid variation of blood fluid balance of the patient. Therefore, recently, a long-time blood purifying therapy, so-called continuous blood purifying therapy (or continuous slow removal) for such patients has been rapidly widespread. In particular, so-called continuous hemodiafiltration (CHDF) using both hemodialysis and hemofiltration has been recognized as being able to minimizes the adverse effect on patients, and therefore this therapy is being widespread drastically. The continuous blood purifying therapy is a method of gradually restoring a patient to a normal condition over such a long time as 24 hours or more, and it is carried out while the flow rate of used dialysate is set to a low flow rate of about one-hundredth to one-tenth of the normal hemodialysis therapy.

The continuous blood purifying therapy is a blood purifying therapy which does not choose the time and place because it does not require any special facilities such as hemodialysis and it can be carried out promptly in the nighttime or in an emergency, and also without any difficulty in finding an installation location thereof even in a place surrounded by a lot of equipment such as an operating room, an ICU or the like. On the other hand, when a patient with severe disease is managed, a doctor is required to stand by at the bedside for 24 hours a day in order to quickly handle a trouble caused by an error in flow rate between dialysate and replacement fluid or the like or quickly exchange blood filtrating tools, so that many facilities avoid the continuous blood purifying therapy due to short of medical staff.
However, a bedside console dedicated to CHDF has been recently developed, and the precision of various kinds of monitors and a flow rate adjusting mechanism has been improved. Therefore, troubles during operation of CHDF can be prevented before it happens, and drastic improvements have been made from the viewpoint of safety of patients and reduction in load imposed on medical staff.

The continuous blood purification apparatus used for the continuous blood purifying therapy which is typified by CHDF is different from the short-time blood purification apparatus in treatment time and peripheral facilities as described above, and thus they are also different in construction. Particularly, the continuous blood purification apparatus is not provided with large-sized auxiliary equipment such as a dialysate preparing apparatus, or a discharging apparatus, and thus it has neither a mechanism for feeding dialysate into inside of the blood purification apparatus nor a mechanism for feeding used dialysate. Accordingly, the continuous blood purification apparatus has been hitherto configured to include a blood purifier and a blood circuit, a dialysate-supply line, a replacement fluid-supply line, and a water-removal line. A dialysate-storage portion, a replacement fluid-storage portion and a used dialysate-container are provided at the outside of the continuous blood purification apparatus. In addition, a roller pump (tubing pump) is used as means for supplying/discharging the dialysate or replacement fluid. Furthermore, a weight measuring system using a weight sensor and a capacity measuring system using a measuring container are used as means for measuring the amount of the dialysate and replacement fluid which is fed into and fed out from a blood circulating path (comprising a blood circuit and a blood purifier).
However, when the roller pump is used for a long-time treatment of 24 hours or more, dialysate is fed by repetitively wringing a dialysate-supply line comprising a tube or the like, and thus the tube is fatigued. Therefore, there is such a problem that the cross-sectional area of the tube varies and thus the amount of fluid flowing in the tube is not constant.
Furthermore, although the precision of the flow rate adjusting mechanism is improved, the weight measuring system has hitherto intermittently measured the fluid weight and thus it is required to perform correction (calibration) such as weight feedback or the like. Therefore, the weight measuring system has such a disadvantage that the device or the circuit is complicated and also correction fails or an alarm is emitted when the device is moved or touched.
The capacity measuring system requires a measuring container every fluid, and measures the capacity while performing calibration of each roller pump (for example, Patent document 1). Therefore, it has a problem that it is necessary to frequently perform calibration to eliminate fluctuation between the pumps.

Patent document 1: JP-A-8-191889 (paragraph number 0014, lines 23-28; paragraph number 0016, lines 23-28)

### DISCLOSURE OF THE INVENTION

### PROBLEM TO BE SOLVED BY THE INVENTION

The present invention has been implemented in view of the foregoing situation, and provides a continuous blood purifying apparatus in which a tube does not fatigue, the error between the flow rate of dialysate and the flow rate of replacement fluid is remarkably small, and calibration of a pump is not required.

### MEANS FOR SOLVING THE PROBLEM

A continuous blood purification apparatus according to the present invention is characterized by comprising a blood purifier and a blood circuit, a dialysate-supply line, a replacement fluid-supply line, and a water-removal line, wherein each of the dialysate-supply line, the replacement fluid-supply line and the water-removal line is provided with a syringe pump; with respect to the dialysate-supply line, the dialysate which is drawn in at once from the dialysate-supply line to the syringe pump can be continuously supplied in small portion to the blood purifier; with respect to the replacement fluid-supply line, the replacement fluid which is drawn in at once from the replacement fluid-supply line to the syringe pump can be continuously supplied in small portion to the venous side or arterial side of the blood circuit; with respect to the water-removal line, the used dialysate which is continuously drawn in from the blood purifier to the syringe pump in small portion can be discharged to the water-removal line at once.
Furthermore, the dialysate-supply line is connected to a dialysate-storage portion at one end thereof and connected to the blood purifier at the other end thereof, branched at some point of the line at which the syringe pump of the dialysate-supply line is provided, provided with a first opening/closing valve at a position between the dialysate-storage portion and the branched portion of the dialysate-supply line, and provided with a second opening/closing valve at a position between the blood purifier and the branched portion of the dialysate-supply line; the replacement fluid-supply line is connected to a replacement fluid-storage portion at one end thereof and connected to the venous side or arterial side of the blood circuit at the other end thereof, branched at some point of the line where the syringe pump of the replacement fluid-supply line is provided, provided with a first opening/closing valve at a position between the replacement fluid-storage portion and the branched portion of the replacement fluid-supply line, and provided with a second opening/closing valve at a position between the blood circuit and the branched portion of the replacement fluid-supply line; the water-removal line is connected to the blood purifier at one end thereof while discharging the used dialysate from the other end thereof, branched at some portion thereof where the syringe pump of the water-removal line is provided, provided with a first opening/closing valve at a position between the blood purifier and the branched portion of the water-removal line, and provided with a second opening/closing valve at a position between the used dialysate discharging side and the branched portion of the water-removal line; (1) when the dialysate is drwn in by the syringe pump provided to the dialysate-supply line, the first opening/closing valve of the dialysate-supply line is opened, the second opening/closing valve of the dialysate-supply line is closed, and when the dialysate is supplied to the blood purifier from the syringe pump provided to the dialysate-supply line, the first opening/closing valve of the dialysate-supply line is closed and the second opening/closing valve of the dialysate-supply line is opened; (2) when the replacement fluid is drawn in by the syringe pump provided to the replacement fluid-supply line, the first opening/closing valve of the replacement fluid-supply line is opened, the second opening/closing valve of the replacement fluid-supply line is closed, and when the replacement fluid is supplied from the syringe pump provided to the replacement fluid-supply line to the venous side or arterial side of the blood circuit, the first opening/closing valve of the replacement fluid-supply line is closed and the second opening/closing valve of the replacement fluid supply-line is opened; and (3) when the used dialysate from the blood purifier is drawn in by the syringe pump provided to the water-removal line, the first opening/closing valve of the water-removal line is opened, the second opening/closing valve of the water-removal line is closed, and when the used dialysate is discharged from the syringe pump provided to the water-removal line, the first opening/closing valve of the water-removal line is closed, and the second opening/closing valve of the water-removal line is opened.

A continuous blood purification apparatus according to the present invention is characterized by comprising a blood purifier, a blood circuit, a dialysate-supply line, a replacement fluid-supply line, and a water-removal line, wherein each of the dialysate-supply line, the replacement fluid-supply line and the water-removal line is provided with a syringe and syringe driving means, and the syringe is secured to the syringe driving means so that the syringe can draw in and discharge the fluid in the line by the syringe driving means.
Syringe state discriminating means may be provided so that a discharge state corresponding to a state that the syringe discharges a predetermined amount of fluid and a drawing state that the syringe draws in a predetermined amount of fluid can be discriminated from each other.
Furthermore, the dialysate supply-line is connected to a dialysate-storage portion at one end thereof and connected to the blood purifier at the other end thereof, branched at some point of the line at which the syringe of the dialysate-supply line is provided, and provided with a first opening/closing valve at a position between the dialysate-storage portion and the branched portion of the dialysate-supply line, and provided with a second opening/closing valve at a position between the blood purifier and the branched portion of the dialysate-supply line; the replacement fluid-supply line is connected to a replacement fluid-storage portion at one end thereof and connected to the venous side or arterial side of the blood circuit at the other end thereof, branched at some point of the line where the syringe of the replacement fluid-supply line is provided, provided with a first opening/closing valve at a position between the replacement fluid-storage portion and the branched portion of the replacement fluid-supply line, and provided with a second opening/closing valve at a position between the blood circuit and the branched portion of the replacement fluid-supply line; the water-removal line is connected to the blood purifier at one end thereof while discharging the used dialysate from the other end thereof, branched at some portion thereof where the syringe of the water-removal line is provided, provided with a first opening/closing valve at a position between the blood purifier and the branched portion of the water-removal line, and provided with a second opening/closing valve at a position between the used dialysate discharging side and the branched portion of the water-removal line; (1) when the dialysate is drawn in by the syringe provided to the dialysate-supply line, the first opening/closing valve of the dialysate-supply line is opened, the second opening/closing valve of the dialysate-supply line is closed, and when the dialysate is supplied to the blood purifier from the syringe provided to the dialysate-supply line, the first opening/closing valve of the dialysate-supply line is closed and the second opening/closing valve of the dialysate-supply line is opened; (2) when the replacement fluid is drawn in by the syringe provided to the replacement fluid-supply line, the first opening/closing valve of the replacement fluid-supply line is opened, the second opening/closing valve of the replacement fluid-supply line is closed, and when the replacement fluid is supplied from the syringe provided to the replacement fluid-supply line to the venous side or arterial side of the blood circuit, the first opening/closing valve of the replacement fluid-supply line is closed and the second opening/closing valve of the replacement fluid-supply line is opened; and (3) when the used dialysate from the blood purifier is drawn in by the syringe provided to the water-removal line, the first opening/closing valve of the water-removal line is opened, the second opening/closing valve of the water-removal line is closed, and when the used dialysate is discharged from the syringe provided to the water-removal line, the first opening/closing valve of the water- removal line is closed, and the second opening/closing valve of the water-removal line is opened.
In the present invention, the used dialysate means fluid flowing from the blood purifier into the water-removal line, and it is used to contain filtered liquid, cleaned liquid, water removed from blood, etc.

The present invention is generally described, however, it can be further understood by referring to some specific embodiments. These embodiments are provided in the specification for the purpose of exemplification, and thus the present invention is not limited to these embodiments unless otherwise specified.

### Effect of the Invention

According to the present invention, the following effect can be expected. That is, the syringe pump is used as the dialysate-pump, the replacement fluid-pump and the water removal-pump, and thus the tube used as each line does not fatigue. Therefore, fluid can be fed and discharged at a stable flow rate. Furthermore, the amounts of the dialysate, the replacement fluid and the used dialysate can be accurately controlled, and thus calibration of the flow rate is not required. Furthermore, the blood circuit is simplified, and the setting of the circuit can be easily performed.

### Brief Description of the Drawings

[Fig. 1] Fig. 1 is a diagram showing a continuous blood purification apparatus according to the present invention.
[Fig. 2] Fig. 2 is a flow diagram when dialysate is drawn in by a dialysate-supply syringe of the continuous blood purification apparatus according to the present invention.
[Fig. 3] Fig. 3 is a flow diagram when continuous hemodialysis (CHD) is executed by using the continuous blood purification apparatus according to the present invention.
[Fig. 4] Fig. 4 is a flow diagram when used dialysate is discharged from a water-removal syringe of the continuous blood purification apparatus according to the present invention.
[Fig. 5] Fig. 5 is a flow diagram when continuous hemofiltration (CHF) is executed by using the continuous blood purification apparatus of the present invention.
[Fig. 6] Fig. 6 is a flow diagram when hemodiafiltration (CHDF) is executed by using the continuous blood purification apparatus according to the present invention.

### Description of Reference Numerals

- 1: blood purifier
- 2: blood circuit
- 21: arterial side blood circuit
- 22: venous side blood circuit
- 3: blood pump
- 4: dialysate-supply line
- 41: dialysate-container
- 42: dialysate-depletion sensor
- 43: dialysate-supply syringe
- 44: dialysate-supply syringe driving means
- 45: dialysate-supply syringe abnormality detecting means
- 5: replacement fluid-supply line
- 51: replacement fluid-container
- 52: replacement fluid-depletion sensor
- 53: replacement fluid-supply syringe
- 54: replacement fluid-supply syringe driving means
- 55: replacement fluid-supply syringe abnormality detecting means
- 6: water-removal line
- 61: used dialysate-container
- 62: water-removal syringe
- 63: water-removal syringe driving means
- 64: water-removal syringe abnormality detecting means
- B1 to B3: branch point
- C1 to C3: drip chamber
- V1 to V7: opening/closing valve

### Best Modes for carrying out the Invention

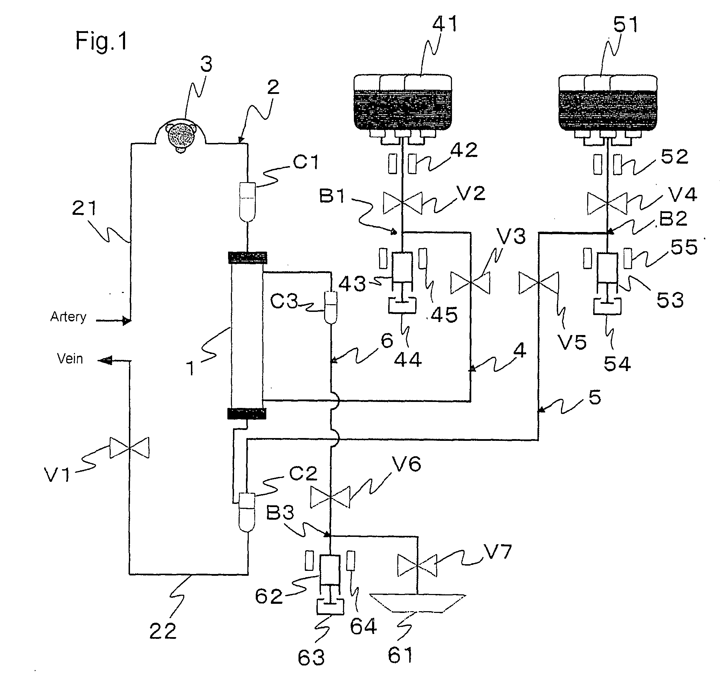

Embodiments of a continuous blood purification apparatus according to the present invention will be described with reference to Figs. 1 to 6.

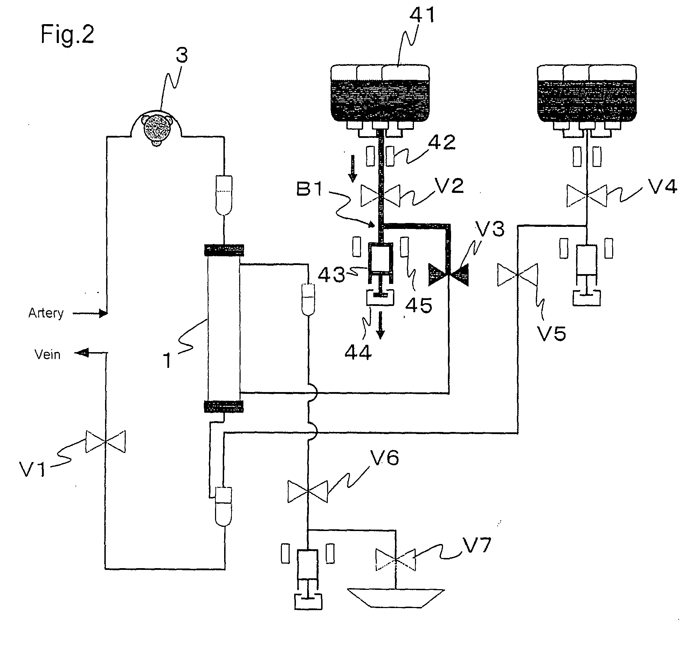

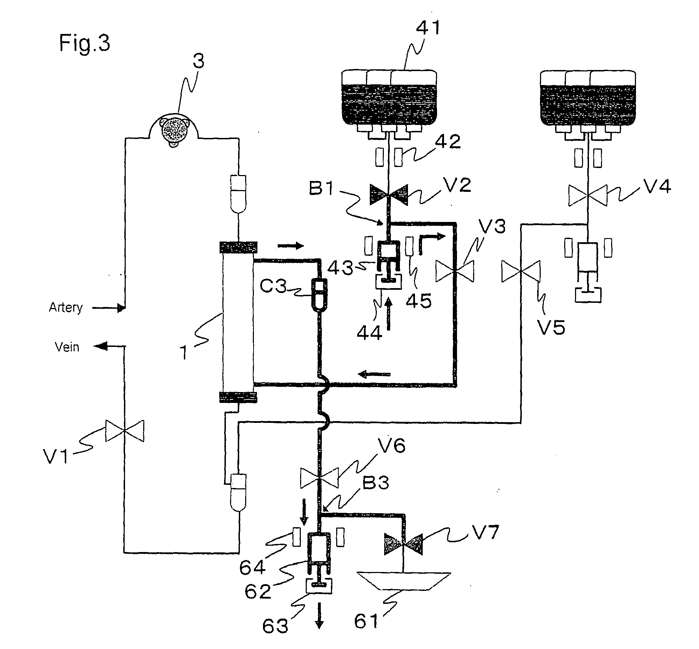

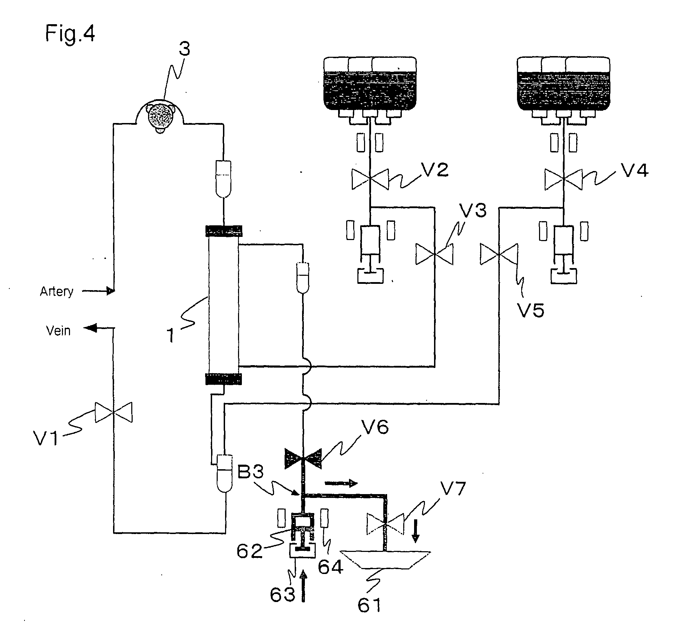

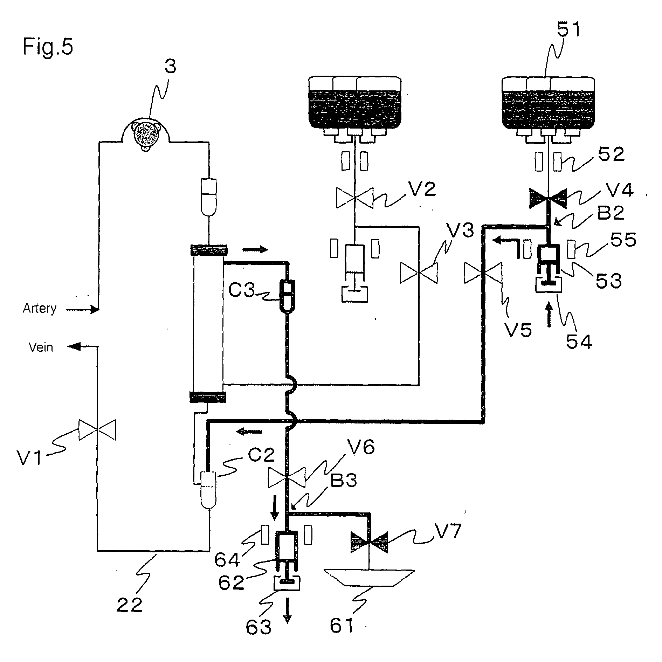

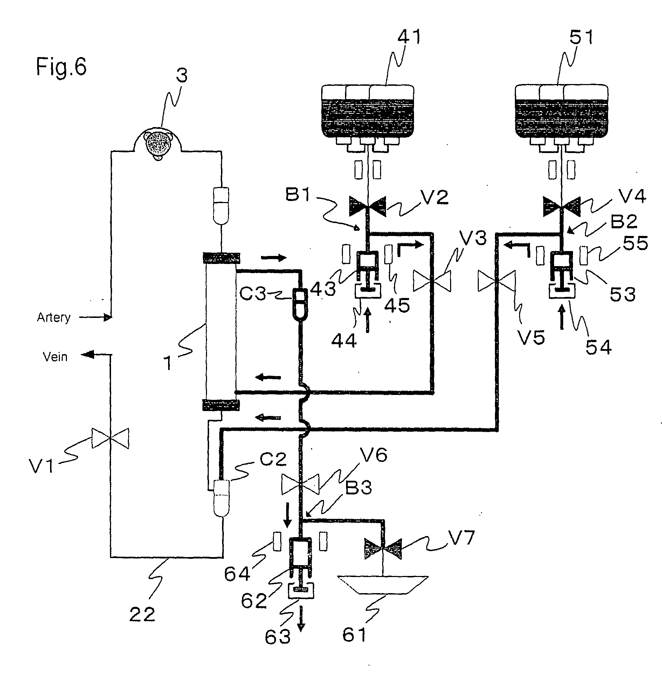

Fig. 1 is a diagram showing the continuous blood purification apparatus according to the present invention, Fig. 2 is a flow diagram when dialysate is drawn in by a dialysate-supply syringe of the continuous blood purification apparatus according to the present invention, Fig. 3 is a flow diagram of continuous hemodialysis (CHD) executed by using the continuous blood purification apparatus according to the present invention, Fig. 4 is a flow diagram when used dialysate is discharged from a water-removal cylinge of the continuous blood purification apparatus according to the present invention, Fig. 5 is a flow diagram of continuous hemofiltration (CHF) executed by using the continuous blood purification apparatus according to the present invention, and Fig. 6 is a flow diagram of continuous hemodiafiltration (CHDF) executed by using the continuous blood purification apparatus according to the present invention. In Figs. 2 to 6, black-painted opening/closing valves are set to a close state.
However, Fig. 2 clearly shows the open/close state of the opening/closing valve of a dialysate-supply line, and does not specify the open/close state of a replacement-fluid supply line and a water-removal line. Furthermore, Fig. 4 clearly shows the open/close state of the opening/closing valve of the water-removal line, and does not specify the open/close state of the opening/closing valves of the dialysate-supply line and the replacement fluid-supply line.
As shown in Fig. 1, the continuous blood purification apparatus of an embodiment is configured to contain a blood purifier 1 and a blood circuit 2, a dialysate-supply line 4, a replacement-fluid supply line 5 and a water-removal line 6, and a dialysate-supply syringe 43, a replacement fluid-supply syringe 53 and a water-removal syringe 62 are provided to the dialysate-supply line 4, the replacement fluid-supply line 5, and the water-removal line 6, respectively.

A syringe pump of this invention contains a syringe and syringe driving means, and it discharges fluid in the syringe to feed the liquid to a circuit and draws in fluid in the syringe to receive the fluid into the circuit.
In this invention, "draw in at once" means a level that blood purification in the blood purifier is not affected, and it represents that a predetermined amount of fluid in the syringe is drawn in at a higher velocity than a feed-out velocity of a predetermined amount of fluid in the syringe. Furthermore, "discharge at once" means a level that blood purification in the blood purifier is not affected, and it represents that a predetermined amount of fluid in the syringe is discharged at a velocity higher than that an inhalation velocity of a predetermined amount of fluid into the syringe.
In this invention, an upstream side indicates a portion nearer to a fluid flow source, and a downstream side indicates a fluid flowing direction. For example, the upstream side of the dialysate-supply line 4 indicates a dialysate-container 41 side, and the downstream side thereof indicates a blood purifier 1 side.

As shown in Fig. 1, the blood purifier 1 is connected to the blood circuit 2, the dialysate supply-line 4 and the replacement fluid-supply line 5. The inside of the blood purifier 1 comprises hollow fiber membrane. When hemodialysis is executed, blood is made to flow into the hollow fiber membrane, and dialysate is made to flow to the outside of the hollow fiber membrane, whereby materials of the blood having small molecular weights can be removed on the basis of the principle of diffusion. When hemofiltration is executed, blood is made to flow into the hollow fiber membrane, and no fluid is made to flow to the outside of the hollow fiber membrane, whereby materials of the blood having larger molecular weights than those in the hemodialysis can be removed by using the pressure difference between the inside and outside of the hollow fiber membrane on the basis of the principle of ultrafiltration. Furthermore, when hemodiafiltration is executed, blood is made to flow into the hollow fiber membrane, dialysate is made to flow to the outside of the hollow fiber membrane, and the pressure difference is applied between the inside and outside of the hollow fiber membrane, whereby unnecessary materials of the blood can be efficiently removed.
The blood flows from an arterial-side blood circuit 21 to the inside of the hollow fiber membrane in the blood purifier 1, and flows in a direction to a venous-side blood circuit 22. On the other hand, the dialysate flows from the dialysate-supply line 4 to the outside of the hollow fiber membrane in the blood purifier 1, and flows in a direction to the water-removal line, that is, in the opposite direction to the blood flow.

The blood circuit 2 represents a passage which contains the inside of the hollow fiber membrane of the blood purifier 1, the arterial-side blood circuit 21, the venous-side blood circuit 22 and parts provided on the circuit such as drip chambers C1, C2, etc. and through which blood passes during blood purification. Tubes of synthetic resin or the like are used as the arterial-side blood circuit 21 and the venous-side blood circuit 22. Preferably, a tube of polyvinyl chloride, a tube of silicone rubber or the like is used. A blood pump 3 for making blood, dialysate, air or the like flow in the circuit is provided on the blood circuit 2. The drip chambers C1 and C2 are provided to remove bubbles in blood, etc.
The arterial-side blood circuit 21 is connectable to an arterial-side puncture needle at one end thereof and also connectable to the blood flow-in port of the blood purifier 1 at the other end thereof. The venous-side blood circuit 22 is connectable to the blood flow-out port of the blood purifier 1 at one end thereof and also connectable to a venous-side puncture needle at the other end thereof. In the case of this embodiment, blood of a patient which is led out from the arterial-side puncture needle passes through the arterial-side blood circuit 21, the drip chamber C1, the blood purifier 1, the drip chamber C2, the venous-side blood circuit 22 and the venous-side puncture needle and then returns into the body of the patient.

The blood pump 3 may be provided at any position insofar as it is located in the blood circuit 2, however, it is preferably provided in the arterial-side blood circuit 22. A roller type, a pulsation type, a centrifugal pump or the like may be used as the blood pump 3.

The dialysate-supply line 4 is connected to a dialysate-container 41 at one end thereof and also connected to the dialysate flow-in port of the blood purifier 1 at the other end thereof. The dialysate-supply line 4 is provided with a first dialysate-supply line opening/closing valve V2 and a second dialysate-supply line opening/closing valve V3 in this order from the upstream side. Furthermore, the dialysate-supply line 4 is provided with a branch point B1 between the first opening/closing valve V2 and the second opening/closing valve V3, a dialysate-supply syringe 43 and dialysate-supply syringe driving means 44 are provided at the branch portion, and dialysate-supply syringe abnormality detecting means 45 is provided as occasion demands. A tube of synthetic resin or the like is used as the dialysate-supply line 4, and preferably polyvinyl chloride tube, a silicone rubber tube or the like is used. Furthermore, a dialysate-depletion sensor 42 is provided as occasion demands. Furthermore, dialysate-supply syringe state discriminating means may be provided as occasion demands.
The dialysate-container 41 may be a container for storing dialysate, and preferably a bag type container such as a dialysate bag or the like is used.
When there occurs such a trouble that the dialysate-container 41 becomes empty or the dialysate-container 41 is not connected to the dialysate-supply line 42 or the like, the dialysate-depletion sensor 42 detects this trouble. Any sensor may be used, and for example, a liquid detection sensor or a bubble detecting sensor is used.
A syringe containing a barrel, a gasket and a plunger is used as the dialysate-supply syringe 43. The tip direction of the dialysate-supply syringe 43 is connected to the branch portion of the dialysate-supply line 4, and the base portion thereof is secured to the dialysate-supply syringe driving means 44. The volume of the dialysate-supply syringe 43 may be arbitrarily determined, and thus the amount of dialysate which can be drawn in/discharged at once can be changed in accordance with the volume of the dialysate-supply syringe 43.
The dialysate-supply syringe driving means 44 can draw in and discharge dialysate to the dialysate-supply syringe 43. For example, driving means for a motor which is rotationally driven so as to press and pull the plunger of the dialysate-supply syringe 43 is used. The flow rate of the dialysate which can be drawn in and discharged is adjusted by the dialysate-supply syringe driving means 44, and the flow rate is preferably adjusted so that the dialysate can be discharged at a flow rate of 0.006 to 6L/h and drawn in at a flow rate which is ten to twenty times as high as that in the discharge operation. More preferably, the dialysate-supply syringe driving means 44 can be electrically operated to automatically draw in dialysate to the dialysate-supply syringe 43 and discharge the dialysate from the dialysate-supply syringe 43.
The dialysate-supply syringe abnormality detecting means 45 detects abnormality when dialysate is not normally drawn in to the dialysate-supply syringe 43 or dialysate is not normally discharged.
The dialysate-supply syringe state discriminating means is the means for discriminating the discharge state and the drawing state of the dialysate-supply syringe 43 from each other. The discharge state of the dialysate-supply syringe 43 represents the state that the dialysate-supply syringe 43 perfectly discharges the dialysate in the syringe or discharges a predetermined amount of dialysate. The drwing state of the dialysate-supply syringe 43 represents the state that the dialysate-supply syringe 43 has perfectly drawn in dialysate to the syringe or has drawn in a predetermined amount of dialysate. The dialysate-supply syringe state discriminating means identifies the state of the syringe, and preferably it may be means that interlocks with the dialysate-supply syringe driving means 44 and identifies the state on the basis of a discharge time of a predetermined amount or means for identifying the state by sensing the position of the gasket of the dialysate-supply syringe 43, the position of the plunger or the flow amount in the dialysate-supply syringe 43.

The replacement fluid-supply line 5 is connected to a replacement fluid-container 51 at one end thereof and also connected to the drip chamber provided to the venous-side blood circuit 22 or provided to the arterial-side blood circuit 21 (in this embodiment, the drip chamber C2 provided to the venous-side blood circuit 22) at the other end thereof. The replacement fluid-supply line 5 is provided with a first replacement fluid-supply line opening/closing valve V4 and a second replacement fluid-supply line opening/closing valve V5 in this order from the upstream side. Furthermore, the replacement fluid-supply line 5 is provided with a branch point B2 between the first opening/closing valve V4 and the second opening/closing valve V5, and a replacement fluid-supply syringe 53 and replacement fluid-supply syringe driving means 54 are provided at the branch portion, and replacement fluid-supply syringe abnormality detecting means 55 is provided as occasion demands. A tube of synthetic resin or the like is used as the replacement fluid-supply line 5, and preferably polyvinyl chloride tube, a silicone rubber tube or the like is used. Furthermore, a replacement fluid-depletion sensor 52 is provided as occasion demands. Furthermore, replacement fluid-supply syringe state discriminating means may be provided as occasion demands.
The replacement fluid-container 51 may be a container for storing replacement fluid, and preferably a bag type container such as a replacement fluid bag or the like is used.
When there occurs such a trouble that the replacement fluid-container 51 becomes empty or the replacement fluid-container 51 is not connected to the replacement fluid-supply line 52 or the like, the replacement fluid-depletion sensor 52 detects this trouble. Any sensor may be used, and for example, a liquid detecting sensor or a bubble detecting sensor is used.
A syringe containing a barrel, a gasket and a plunger is used as the replacement fluid-supply syringe 53. The tip direction of the replacement fluid-supply syringe 53 is connected to the branch portion of the replacement fluid-supply line 5, and the base portion thereof is secured to the replacement fluid-supply syringe driving means 54. The volume of the replacement fluid-supply syringe 53 may be arbitrarily determined, and thus the amount of replacement fluid which can be drawn in/ discharged at once can be changed in accordance with the volume of the replacement fluid-supply syringe 53.
The replacement fluid-supply syringe driving means 54 can draw in and discharge replacement fluid to the replacement fluid-supply syringe 53. For example, driving means for a motor which is rotationally driven so as to press and pull the plunger of the replacement fluid-supply syringe 53 is used. The flow rate of the replacement fluid which can be drawn in and discharged is adjusted by the replacement fluid-supply syringe driving means 54, and the flow rate is preferably adjusted so that the replacement fluid can be discharged at a flow rate of 0.001 to 6L/h and drawn in at a flow rate which is ten to twenty times as high as that in the discharge operation. More preferably, the replacement fluid-supply syringe driving means 54 can be electrically operated to automatically draw in replacement fluid to the replacement fluid-supply syringe 53 and discharge the replacement fluid from the replacement fluid-supply syringe 53.
The replacement fluid-supply syringe abnormality detecting means 55 detects abnormality when replacement fluid is not normally drawn in to the replacement fluid-supply syringe 53 or replacement fluid is not normally discharged.
The replacement fluid-supply syringe state discriminating means is the means for discriminating the discharge state and the drawing state of the replacement fluid-supply syringe 53 from each other. The discharge state of the replacement fluid-supply syringe 53 represents the state that the replacement fluid-supply syringe 53 perfectly discharges the replacement fluid in the syringe or discharges a predetermined amount of replacement fluid. The drawing state of the replacement fluid-supply syringe 53 represents the state that the replacement fluid-supply syringe 53 has perfectly drawn in replacement fluid to the syringe or has drawn in a predetermined amount of replacement fluid. The replacement fluid-supply syringe state discriminating means identifies the state of the syringe, and preferably it may be means that interlocks with the replacement fluid-supply syringe driving means 54 and identifies the state on the basis of a discharge time of a predetermined amount or means for identifying the state by sensing the position of the gasket of the replacement fluid-supply syringe 53, the position of the plunger or the flow amount in the replacement fluid supply syringe 53.

The water-removal line 6 is connected to the dialysate flow-out port of the blood purifier 1 at one end thereof and discharges the used dialysate from the other end thereof. Preferably, the used dialysate-container 61 is connected to the other end of the water-removal line 6. The water-removal line 6 is provided with a first water-removal line opening/closing valve V6 and a second water-removal line opening/closing valve V7 in this order from the upstream side. A drip chamber C3 may be provided as occasion demands. Furthermore the water-removal line 6 is provided with a branch point B3 between the first opening/closing valve V6 and the second opening/closing valve V7, a water-removal syringe 62 and water-removal syringe driving means 63 are provided at the branch portion, and water-removal syringe abnormality detecting means 64 is provided as occasion demands. A tube of synthetic resin or the like is used as the water-removal line 6, and preferably a polyvinyl chloride tube, a silicone rubber tube or the like is used. Furthermore, water-removal syringe state discriminating means may be provided as occasion demands.
The used dialysate-container 61 is a container from which the used dialysate is discharged.
A syringe containing a barrel, a gasket and a plunger is used as the water-removal syringe 62. The tip direction of the water-removal syringe 62 is connected to the branch portion of the water-removal line 6, and the base portion thereof is secured to the water-removal syringe driving means 63. The volume of the used water-removal syringe 62 may be arbitrarily determined, and thus the amount of the used dialysate which can be drawn in/discharged at once can be changed in accordance with the volume of the water-removal syringe 62.
The water-removal syringe driving means 63 can draw in and discharge the used dialysate to the water-removal syringe 62. For example, driving means for a motor which is rotationally driven so as to press and pull the plunger of the water-removal syringe 62 is used. The flow rate of the used dialysate which can be drawn in and discharged is adjusted by the water-removal syringe driving means 63, and the flow rate is preferably adjusted so that the used dialysate can be drawn in at a flow rate of 0.001 to 6L/h and discharged at a flow rate which is ten to twenty times as high as that in the inhalation operation.
The water-removal syringe abnormality detecting means 64 detects abnormality when used dialysate is not normally drawn in to the water-removal syringe 62 or used dialysate is not normally discharged.
The water-removal syringe state discriminating means is the means for discriminating the discharge state and the drawing state of the water-removal syringe 62 from each other. The discharge state of the water-removal syringe 62 represents the state that the water-removal syringe 62 perfectly discharges the used dialysate in the syringe or discharges a predetermined amount of used dialysate. The drawing state of the water-removal syringe 62 represents the state that the water-removal syringe 62 has perfectly drawn in the used dialysate to the syringe or has drawn in a predetermined amount of used dialysate. The water-removal syringe state discriminating means identifies the state of the syringe, and preferably it may be means that interlocks with the water-removal syringe driving means 63 and identifies the state on the basis of a discharge time of a predetermined amount or means for identifying the state by sensing the position of the gasket of the water-removal syringe 62, the position of the plunger or the flow amount in the water-removal syringe 62.

Any valve may be adopted as the opening/closing valves V1 to V7 (the blood circuit opening/closing valve v1, the first dialysate-supply line opening/closing valve V2, the second dialysate-supply line opening/closing valve V3, the first replacement fluid-supply line opening/closing valve V4, the second replacement fluid-supply line opening/closing valve V5, the first water-removal line opening/closing valve V6 and the second water-removal line opening/closing valve V7) insofar as the valve functions as an opening/closing valve. It is preferable that the opening/closing state can be controlled in an electrical operation style and the opening/closing operation can be automatically performed by the syringe state discriminating means provided to each line.

Next, use of the continuous blood purification apparatus according to the present invention will be described.
A case where the continuous hemodialysis is executed by using the continuous blood purification apparatus according to the present invention will be described hereunder.
From the close state of the opening/closing valves V1 to V7, the first dialysate-supply line opening/closing valve V2 is opened, and dialysate from the dialysate-container 41 is drawn in to the dialysate-supply syringe 43 by the dialysate-supply syringe driving means 44 (the amount of the dialysate to be drawn in can be arbitrarily determined) as shown in Fig. 2. Preferably, the dialysate is drawn in to the dialysate supply syringe 43 at once(the driving time is set to 2 to 3 seconds).
Subsequently, as shown in Fig. 3, the blood circuit opening/closing valve V1 is opened, and the blood pump 3 is driven to circulate blood in the blood circulation passage. Thereafter, the first dialysate-supply line opening/closing valve V2 is closed, the second dialysate-supply line opening/closing valve V3 and the first water-removal line opening/closing valve V6 are opened, and dialysate is continuously supplied at a low flow rate from the dialysate-supply syringe 43 into the blood purifier 1 by the dialysate-supply syringe driving means 44 and the water-removal syringe driving means 63. At the same time, the used dialysate is continuously drawn in at substantially the same flow rate as the dialysate supply syringe 43 from the blood purifier 1 to the water-removal syringe 62.
Subsequently, as shown in Fig. 4, the first water-removal line opening/closing valve V6 is closed, the second water-removal line opening/closing valve V7 is opened, and the used dialysate is discharged from the water-removal syringe 62 into the used dialysate container 61 by the water-removal syringe driving means 63. At this time, preferably, the second dialysate-supply line opening/closing valve V3 is closed simultaneously with the closing of the first water-removal line opening/closing valve V6, the first dialysate-supply line opening/closing valve V2 is opened simultaneously with the opening of the second water-removal line opening/closing valve V7, the dialysate is drawn in from the dialysate container 41 to the dialysate supply syringe 43 at once by the dialysate-supply syringe driving means 44 and the water-removal line syringe driving means 63, and the used dialysate is discharged from the water-removal syringe 62 into the used dialysate-container 61 at once (the diving time is set to 2 to 3 seconds). Subsequently, the same operation may be repeated in the dialysate-supply line 4 while blood is circulated in the blood circuit 2.

A case where the continuous hemofiltration is executed by using the continuous blood purification apparatus according to the present invention will be described hereunder.
From the close state of the opening/closing valves V1 to V7, the first replacement fluid-supply line opening/closing valve V4 is opened, and the replacement fluid is drawn in from the replacement fluid-container 51 to the replacement fluid-supply syringe 53 by the replacement fluid-supply syringe driving means 54 (the amount of the replacement fluid to be drawn in can be arbitrarily determined). The same operation as the operation executed on the dialysate-supply line of Fig. 2 may be executed on the replacement fluid-supply line. Preferably, the replacement fluid is drawn in to the replacement fluid-supply syringe 53 at once (the driving time is set to 2 to 3 seconds).
Subsequently, as shown in Fig. 5, the blood circuit opening/closing valve V1 is opened, and the blood pump 3 is driven to circulate blood in the blood circulation passage. Thereafter, the first replacement fluid-supply line opening/closing valve V4 is closed, the second replacement fluid-supply line opening/closing valve V5 and the first water-removal line opening/closing valve V6 are opened, and replacement fluid is continuously supplied at a low flow rate from the replacement fluid-supply syringe 53 to the venous-side blood circuit 22 by the replacement fluid-supply syringe driving means 54 and the water-removal syringe driving means 63. At the same time, the used dialysate is continuously drawn in at a low flow rate (which is not less than the flow rate of the replacement fluid-supply syringe 53 and an excess amount is determined by the removed water amount) from the blood purifier 1 to the water-removal syringe 62.
Subsequently, as shown in Fig. 4, the first water-removal line opening/closing valve V6 is closed, the second water-removal line opening/closing valve V7 is opened, and the used dialysate is discharged from the water-removal syringe 62 into the used dialysate-container 61 by the water-removal syringe driving means 63. At this time, preferably, the second replacement fluid-supply line opening/closing valve V5 is closed simultaneously with the closing of the first water-removal line opening/closing valve V6, the first replacement fluid-supply line opening/closing valve V4 is opened simultaneously with the opening of the second water-removal line opening/closing valve V7, the dialysate is drawn in at once from the replacement fluid-container 51 to the replacement fluid-supply syringe 53 by the replacement fluid-supply syringe driving means 54 and the water-removal line syringe driving means 63, and the used dialysate is discharged from the water-removal syringe 62 into the used dialysate-container 61 at once (the driving time is set to 2 to 3 seconds). Subsequently, the same operation may be repeated in the replacement fluid-supply line 5 while blood is circulated in the blood circuit 2.

A case where the continuous hemodiafiltration is executed by using the continuous blood purification apparatus according to the present invention will be described hereunder.
From the close state of the opening/closing valves V1 to V7, the first dialysate-supply line opening/closing valve V2 and the first replacement fluid-supply line opening/closing valve V4 are opened, the dialysate is drawn in from the dialysate-container 41 to the dialysate-supply syringe 43 by the dialysate-supply syringe driving means 44, and the replacement fluid is drawn in from the replacement fluid-container 51 to the replacement fluid-supply syringe 53 by the replacement fluid-supply syringe driving means 54 (the amounts of the dialysate and the replacement fluid to be drawn in can be arbitrarily determined) . The same operation as the operation executed on the dialysate-supply line of Fig. 2 may be executed on the dialysate-supply line and the replacement fluid-supply line. Preferably, the dialysate is drawn in to the dialysate-supply syringe 43 at once (the driving time is set to 2 to 3 seconds), and the replacement fluid is drawn in to the replacement fluid-supply syringe 53 at once (the driving time is set to 2 to 3 seconds).
Subsequently, as shown in Fig. 6, the blood circuit opening/closing valve V1 is opened, and the blood pump 3 is driven to circulate blood in the blood circulation passage. Thereafter, the first dialysate-supply line opening/closing valve V2 and the first replacement fluid-supply line opening/closing valve V4 are closed, the second dialysate-supply line opening/closing valve V3, the second dialysate-supply line opening/closing valve V5 and the first water-removal line opening/closing valve V6 are opened, the dialysate is continuously supplied at a low flow rate from the dialysate-supply syringe 43 into the blood purifier 1 by the dialysate-supply syringe driving means 44, the replacement fluid-supply syringe driving means 54 and the water-removal syringe driving means 63, and the replacement fluid is continuously supplied at a low flow rate from the replacement fluid-supply syringe 53 to the venous-side blood circuit 22. At the same time, the used dialysate is continuously drawn in at a low flow rate (which is not less than the total flow rate of the dialysate-supply syringe 43 and the replacement fluid-supply syringe 53, and an excessive amount is determined by the removed water amount) from the blood purifier 1 to the water-removal syringe 62.
Subsequently, as shown in Fig. 4, the first water-removal line opening/closing valve V6 is closed, the second water-removal line opening/closing valve V7 is opened, and the used dialysate is discharged from the water-removal syringe 62 into the used dialysate-container 61 by the water-removal syringe driving means 63. At this time, preferably, the second dialysate-supply line opening/closing valve V3 and the second replacement fluid-supply line opening/closing valve V5 are closed simultaneously with the closing of the first water-removal line opening/closing valve V6, the first dialysate-supply line opening/closing valve V2 and the first replacement fluid-supply line opening/closing valve V4 are opened simultaneously with the opening of the second water-removal line opening/closing valve V7, the dialysate is drawn in at once from the dialysate-container 41 to the dialysate-supply syringe 43 by the dialysate-supply syringe driving means 44, the replacement fluid-supply syringe driving means 54 and the water-removal line syringe driving means 63, and the replacement fluid is drawn in at once from the replacement fluid-container 51 to the replacement fluid-supply syringe 53. At the same time, the used dialysate is discharged from the water-removal syringe 62 into the used dialysate-container 61 at once (the driving time is set to 2 to 3 seconds). Subsequently, the same operation may be repeated in the dialysate-supply line 4 and the replacement fluid-supply line 5 while blood is circulated in the blood circuit 2.

### Industrial Applicability

As described above, the continuous blood purification apparatus in which the tube does not fatigue, the error of the dialysate flow rate and the replacement fluid flow rate is extremely small, and also the calibration of the pump is not required can be attained by using the syringe pump. Furthermore, the continuous blood purifying therapy such as the continuous hemodialysis, the continuous hemofiltration, or the continuous hemodiafiltration can be more safely implemented with reducing the load imposed on medical staff by the continuous blood purification apparatus according to the present invention.

## Claims

1. A continuous blood purification apparatus comprising: a blood purifier and a blood circuit, a dialysate-supply line, a replacement fluid-supply line, and a water-removal line, wherein each of the dialysate-supply line, the replacement fluid-supply line and the water-removal line is provided with a syringe pump; with respect to the dialysate-supply line, the dialysate which is drawn in at once from the dialysate-supply line to the syringe pump can be continuously supplied in small portion to the blood purifier; with respect to the replacement fluid-supply line, the replacement fluid which is drawn in at once from the replacement fluid-supply line to the syringe pump can be continuously supplied in small portion to the venous side or arterial side of the blood circuit; with respect to the water-removal line, the used dialysate which is continuously drawn in from the blood purifier to the syringe pump in small portion can be discharged to the water-removal line at once.

2. The continuous blood purification apparatus according to claim 1, wherein the dialysate-supply line is connected to a dialysate-storage portion at one end thereof and connected to the blood purifier at the other end thereof, branched at some point of the line at which the syringe pump of the dialysate-supply line is provided, provided with a first opening/closing valve at a position between the dialysate-storage portion and the branched portion of the dialysate-supply line, and provided with a second opening/closing valve at a position between the blood purifier and the branched portion of the dialysate-supply line;
wherein the replacement fluid-supply line is connected to a replacement fluid-storage portion at one end thereof and connected to the venous side or arterial side of the blood circuit at the other end thereof, branched at some point of the line where the syringe pump of the replacement fluid-supply line is provided, provided with a first opening/closing valve at a position between the replacement fluid-storage portion and the branched portion of the replacement fluid-supply line, and provided with a second opening/closing valve at a position between the blood circuit and the branched portion of the replacement fluid-supply line;
wherein the water-removal line is connected to the blood purifier at one end thereof while discharging the used dialysate from the other end thereof, branched at some portion thereof where the syringe pump of the water-removal line is provided, provided with a first opening/closing valve at a position between the blood purifier and the branched portion of the water-removal line, and provided with a second opening/closing valve at a position between the used dialysate discharging side and the branched portion of the water-removal line;
(1) when the dialysate is drawn in by the syringe pump provided to the dialysate-supply line, the first opening/closing valve of the dialysate-supply line is opened, the second opening/closing valve of the dialysate-supply line is closed, and when the dialysate is supplied to the blood purifier from the syringe pump provided to the dialysate-supply line, the first opening/closing valve of the dialysate-supply line is closed and the second opening/closing valve of the dialysate-supply line is opened;
(2) when the replacement fluid is drawn in by the syringe pump provided to the replacement fluid-supply line, the first opening/closing valve of the replacement fluid-supply line is opened, the second opening/closing valve of the replacement fluid-supply line is closed, and when the replacement fluid is supplied from the syringe pump provided to the replacement fluid-supply line to the venous side or arterial side of the blood circuit, the first opening/closing valve of the replacement fluid-supply line is closed and the second opening/closing valve of the replacement fluid-supply line is opened; and
(3) when the used dialysate from the blood purifier is drawn in by the syringe pump provided to the water-removal line, the first opening/closing valve of the water-removal line is opened, the second opening/closing valve of the water-removal line is closed, and when the used dialysate is discharged from the syringe pump provided to the water-removal line, the first opening/closing valve of the water-removal line is closed, and the second opening/closing valve of the water-removal line is opened.

3. A continuous blood purification apparatus, comprising: a blood purifier, a blood circuit, a dialysate-supply line, a replacement fluid-supply line, and a water-removal line, wherein each of the dialysate-supply line, the replacement fluid-supply line and the water-removal line is provided with a syringe and syringe driving means, and the syringe is secured to the syringe driving means so that the syringe can draw in and discharge the fluid in the line by the syringe driving means.

4. The continuous blood purification apparatus according to claim 3, wherein syringe state discriminating means is provided so that a discharging state corresponding to a state that the syringe discharges a predetermined amount of fluid and a drawing state that the syringe draws in a predetermined amount of fluid can be discriminated from each other.

5. The continuous blood purification apparatus according to claim 3 or 4, wherein the dialysate-supply line is connected to a dialysate-storage portion at one end thereof and connected to the blood purifier at the other end thereof, branched at some point of the line at which the syringe of the dialysate-supply line is provided, provided with a first opening/closing valve at a position between the dialysate-storage portion and the branched portion of the dialysate-supply line, and provided with a second opening/closing valve at a position between the blood purifier and the branched portion of the dialysate-supply line;
wherein the replacement fluid-supply line is connected to a replacement fluid-storage portion at one end thereof and connected to the venous side or arterial side of the blood circuit at the other end thereof, branched at some point of the line where the syringe of the replacement fluid-supply line is provided, provided with a first opening/closing valve at a position between the replacement fluid-storage portion and the branched portion of the replacement-fluid supply line, and provided with a second opening/closing valve at a position between the blood circuit and the branched portion of the replacement fluid-supply line;
wherein the water-removal line is connected to the blood purifier at one end thereof while discharging the used dialysate from the other end thereof, branched at some portion thereof where the syringe of the water-removal line is provided, provided with a first opening/closing valve at a position between the blood purifier and the branched portion of the water-removal line, and provided with a second opening/closing valve at a position between the used dialysate discharging side and the branched portion of the water-removal line;
(1) when the dialysate is drawn in by the syringe provided to the dialysate-supply line, the first opening/closing valve of the dialysate-supply line is opened, the second opening/closing valve of the dialysate-supply line is closed, and when the dialysate is supplied to the blood purifier from the syringe provided to the dialysate-supply line, the first opening/closing valve of the dialysate-supply line is closed and the second opening/closing of the dialysate-supply line is opened;
(2) when the replacement fluid is drawn in by the syringe provided to the replacement fluid-supply line, the first opening/closing valve of the replacement fluid-supply line is opened, the second opening/closing valve of the replacement fluid-supply line is closed, and when the replacement fluid is supplied from the syringe provided to the replacement fluid-supply line to the venous side or arterial side of the blood circuit, the first opening/closing valve of the replacement fluid-supply line is closed and the second opening/closing valve of the replacement fluid-supply line is opened; and
(3) when the used dialysate from the blood purifier is drawn in by the syringe provided to the water-removal line, the first opening/closing valve of the water-removal line is opened, the second opening/closing valve of the water-removal line is closed, and when the used dialysate is discharged from the syringe provided to the water-removal line, the first opening/closing valve of the water-removal line is closed, and the second opening/closing valve of the water-removal line is opened.
